# EUROPEAN PATENT APPLICATION

(11) **EP 4 545 019 A1**
(43) Date of publication of application: **30.04.2025**
(21) Application number: 24208179.2
(22) Date of filing: 22.10.2024
(51) Int. Cl.: A61B 17/16, F16D 1/116, A61B 17/00

(54) **MODULAR INSTRUMENT**

(30) Priority: 24.10.2023 US 202363545423 P
(71) Applicant: Howmedica Osteonics Corp., Mahwah, NJ 07430 (US)
(72) Inventor: CROUS, Heinrich George, Killeagh, P36F291 (IE)
(74) Representative: Keller Schneider Patentanwaltsgesellschaft mbH

(57) **Abstract**

A flexible modular instrument (100) includes a shaft (110) and an extension piece (130). The shaft (110) includes an interior segment (112) having a first outer cross-sectional dimension and an end segment (120) extending from the interior segment (112) having a second outer cross-sectional dimension less than the first outer cross-sectional dimension. The interior segment (112) includes a radial protrusion (118a, 118b) adjacent to the end segment (120) such that the radial protrusion (118a, 118b) extends further from a central longitudinal axis (X) of the shaft (110) than an outer surface of the interior segment (112), wherein the first and second outer cross-sectional dimensions are measured orthogonally relative to the central longitudinal axis (X). The extension piece (130) includes an attachment portion (132) removably engageable with the end segment (120) of the shaft (110). The attachment portion (132) includes an opening (134) to receive the end segment (120), the opening (134) being defined in part by a pair of arms (136a, 136b), each arm of the pair of arms (136a, 136b) including a projection (138a, 138b) into the opening (134).

## Description

### CROSS-REFERENCE TO RELATED APPLICATIONS

This application claims the benefit of the filing date of the of United States Provisional Patent Application No. 63/545,423 filed on October 24, 2023, the disclosure of which is hereby incorporated by reference

### BACKGROUND

Certain rotary cutting tools include a driving component and a cutting component which is driven, e.g., rotated, by the driving component to cut materials and anatomical structures such as bone. The cutting component of such tools is typically either a larger singular component, which cannot accommodate different sizes or cutting edges, or can be made up of separate components. When separate components are used, such components can be coupled via standardized instrumentation connections such as an AO coupling connection fabricated using conventional techniques and requiring several individual components such as a main housing body, stainless steel ball bearings, springs and pull buttons interacting in combination to perform the cutting function. In other words, conventional connections are often created using complex assemblies involving a large number of components, and are therefore cumbersome to assemble and use.

Accordingly, a tool or instrument having a structure with a shaft and a cutting component that improves stability and convenience of use of the instrument is desirable to improve the overall efficacy of surgical procedures.

### BRIEF SUMMARY

The medical instrument disclosed herein provides a cutting component attached to a shaft component using a simple, convenient and stable connection between two parts that can easily be coupled and decoupled from one another. The structure of the respective parts may be formed by additively manufacturing one piece and subtractively manufacturing the other piece to form the proper structure for conveniently forming a stable connection between the two pieces and allowing for easy disengagement of the same. While the above represents one arrangement, it should be appreciated that in variations, both pieces may be additively manufactured. The contemplated methods of manufacture of the medical instrument leverage the best of multiple manufacturing methods to provide a structure with two components where the shaft component may be made through a subtractive method for purposes of economy, while the intricacies of the cutting component are well suited for manufacture through additive manufacturing methods. The cutting component of the medical instrument design includes a modular connection with flexible features to create an interlocking fixation between two components and to allow interchangeability with other attachable components. Specifically, the medical instrument includes a male and female connection between two components where the male portion is located on the shaft piece and the female portion is located on the cutting component piece. The flexible female portion of the connection does not require any additional machining or components after initial manufacture.

According to a first aspect of the disclosure, an instrument may include a shaft and an extension piece. The shaft may include an interior segment and an end segment extending from the interior segment. The interior segment may have a first outer cross-sectional dimension and the end segment may have a second outer cross-section dimension less than the first outer cross-sectional dimension. The interior segment may include a radial protrusion adjacent to the end segment such that the radial protrusion extends further from a central longitudinal axis of the shaft than an outer surface of the interior segment, wherein the first and second outer cross-sectional dimensions are measured orthogonally relative to the central longitudinal axis. The extension piece may include an attachment portion removably engageable with the end segment of the shaft. The attachment portion may include an opening to receive the end segment and the opening may be defined in part by a pair of arms. Each are of the pair of arms may include a projection into the opening. The pair of arms may be elastically deformable such that when the shaft is inserted into the opening of the extension piece and the radial protrusion passes the projections of the pair of arms, the pair of arms are pushed apart from a neutral position. When the radial protrusion is in between the projections and an interior end of the opening, the projections may be in the neutral position and prevent back out of the shaft from the extension piece.

Further in instrument of the first aspect of the disclosure, the extension piece may include a cutting edge separate from the attachment portion. The extension piece may be formed monolithically. The pair of arms may be positioned opposite each other and may be separated by the opening. The projection of a first arm of the pair of arms may be disposed opposite the projection of a second arm of the pair of arms such that the projections of the first and second arms are separated by the opening and face each other. The attachment portion may further include a pair of tabs that further define the opening. The pair of tabs may be positioned opposite each other and separated by the opening, the pair of tabs separating the pair of arms. The pair of tabs may be offset from the pair of arms by ninety degrees around the opening formed by the attachment portion. The pair of tabs may be positioned around the opening such that when a radially inward pressure is applied to the pair of tabs, the pair of tabs move inward and cause a radially outward deformation of the pair of arms.

Further in the instrument of the first aspect of the disclosure, when the radial protrusion is in between the projections and the interior end of the opening, the extension piece may be rotationally fixed to the shaft. The end segment may include a neck extending from the interior segment having outer cross-sectional dimensions smaller than the first outer cross-sectional dimension of the interior segment. The attachment portion of the extension piece may define an opening formed by surfaces that complement surfaces of the neck of the end segment. A cross-section of the neck of the end segment along a plane orthogonal to the central longitudinal axis may define an oblong shape such that when the neck is received within the bore of the extension piece, rotational movement of the extension piece relative to the end segment is prevented. The end segment may include a head extending from the neck and the head may be cylindrical. The interior segment may by cylindrical and may define a pair of opposing recesses at an end of the interior segment adjacent to the radial protrusion. The instrument may be provided in a kit along with a second extension piece, wherein the second extension piece may be adapted for removable engagement with the shaft. The kit may further include a plurality of the extension pieces, wherein each of the plurality of extension pieces may define a cutting element different from another extension piece, and each extension piece may be interchangeably coupleable to the shaft. The second extension piece may be different from the first extension piece. The shaft of the instrument may be subtractively manufactured. The extension piece may be additively manufactured. The interior segment of the shaft may define an opposing pair of recesses defined radially inward of the radial protrusion. The radial protrusion may include an opposing a pair of lips on the interior segment and the opposing pair of lips may be adjacent to the end segment. Each of the opposing pair of lips may be adjacent to one of a corresponding opposing pair of recesses defined on the interior segment such that each one of the pair of lips extends radially outward beyond the corresponding recess.

According to a second aspect of the disclosure, an instrument may include a shaft and an extension piece. The shaft may include an end segment. The extension piece may include an attachment portion removably engageable with the end segment of the shaft. The attachment portion may include an opening to receive the end segment. The opening may be defined by a pair of flexible arms and a pair of flexible tabs. The pair of flexible arms may be opposite each other and may be separated by the pair of flexible tabs. Each arm of the pair of arms may include a projection into the opening. The pair of arms may be elastically deformable such that when the end segment of the shaft is inserted into the opening of the extension piece, the pair of arms prevent back out of the shaft from the extension piece. The end segment may be removable from within the opening by pressing the pair of tabs inward toward the center of the opening, thereby pushing the pair of arms away from each other.

Further in the instrument according to the second aspect of the disclosure, the extension piece may be engaged with the shaft, the extension piece is axially and rotationally fixed to the shaft. The end segment may include a neck defining a non-circular cross-section in a plane orthogonal to the central longitudinal axis. The attachment portion may define a bore sized and shaped to mate with the neck of the end segment when the shaft is engaged with the extension piece. The shaft may define a pair of opposing recessed surfaces. Each of the opposing recessed surfaces may include a corresponding lip forming a pair of opposing lips configured to engage with the pair of opposing arms of the extension piece when the extension piece is engaged with the shaft. Each arm of the pair of opposing arms may be configured to be seated on a side of a corresponding lip of the pair of lips opposite a free end of the end segment when the end segment is inserted into the opening of the extension piece. The pair of opposing arms may be seated adjacent the pair of opposed recessed surfaces when the end segment is inserted into the opening of the extension piece to prevent back out of the shaft. When the pair arms is in a non-deformed state, the projections of the pair of arms may be closer to the central longitudinal axis than an outer surface of each lip on the pair of recessed surfaces. When the pair of arms are in a deformed state passing over the respective lips of the shaft, a majority of the projections of the pair of arms may be farther from the central longitudinal axis than a radially outward extent of the outer surface of each lip on the pair of recessed surfaces. The pair of opposing arms may define a nondeformed state when the pair of arms are at rest and a deformed state when the pair of tabs are pressed inward, and the shaft may be axially translatable relative to the extension piece when the pair of arms is in the deformed state.

According to a method of using the instrument of the second aspect of the disclosure, the method may include driving the shaft when the extension piece is engaged to the shaft such that the shaft and the extension piece rotate about a single central longitudinal axis of the shaft, and cutting bone with a cutting edge of the extension piece. The method may further include pressing the pair of flexible tabs to deform the attachment portion of the extension piece, and disengaging the extension piece from the shaft. The extension piece of the instrument may be a first extension piece, and the method may further include subsequent to disengaging the first extension piece from the shaft, engaging a second extension piece with the shaft, wherein the second extension piece may include an attachment portion removably engageable with the end segment of the shaft, the attachment portion including an opening to receive the end segment, the opening being defined by a pair of flexible arms and a pair of flexible tabs, the pair of flexible arms being opposite each other and separated by the pair of flexible tabs, each arm of the pair of arms including a projection into the opening.

According to a third aspect of the disclosure, a method of using an instrument may include inserting a first end of a shaft into an internal opening of an extension piece, the insertion step comprising; positioning the shaft so that a radial protrusion of the shaft is on a first side of a projection extending radially inward into the internal opening of the extension piece; advancing the radial protrusion along a ramp formed by a first surface of the projection as the extension piece deforms; and passing the radial protrusion of the shaft from a first side of a second surface of the projection to a second opposing side of the surface of the projection so that the extension piece return to a non-deformed state.

Further in the method according to the third aspect of the disclosure, after the step of passing the radial protrusion of the shaft from the first side of the second surface of the projection to the second opposing side, the shaft may be prevented from being removed from the extension piece. The method may further include rotationally aligning the shaft relative to the extension piece such that elongate sides of a neck portion on the shaft align with elongate sides elongate sides formed by the opening defined by the extension piece. The method may further include inwardly pressing an opposing pair of tabs included on the extension piece to elastically deform the extension piece and the opening defined by the extension piece. The method may further include after the step of inwardly pressing the opposing pair of tabs, withdrawing the shaft from the extension piece to decouple the shaft from the extension piece while the extension piece is elastically deformed. The extension piece may be a first extension piece and the method may further include removing the first extension piece from the shaft and inserting the shaft into an opening defined by a second extension piece to couple the shaft to a second extension piece.

### BRIEF DESCRIPTION OF THE DRAWINGS

FIG. 1 is a perspective view of a modular instrument according to an embodiment of the disclosure.
FIG. 2 is a perspective view of decoupled components of the modular instrument of FIG. 1.
FIG. 3 is an isolated view of a shaft of the modular instrument of FIG. 1.
FIG. 4 is a side view of a first end of the shaft of FIG. 3.
FIGS. 5-6 are isolated perspective views of an extension piece of the modular instrument of FIG. 1.
FIG. 7 is a top view of the extension piece of FIG. 6.
FIG. 8 is a cross-sectional view of the modular instrument of FIG. 1 in a decoupled configuration.
FIG. 9 is a close-up partial view of the cross-section of the connection mechanism of the modular instrument of FIG. 8.
FIG. 10 is a close-up partial view of the cross-section of the connection mechanism of the modular instrument of FIG. 1 in a coupled configuration.

### DETAILED DESCRIPTION

As used herein, the terms "about," "generally," "approximately," and "substantially" are intended to mean that slight deviations from absolute are included within the scope of the term so modified. However, unless otherwise indicated, the lack of any such terms should not be understood to mean that such slight deviations from absolute are not included within the scope of the term so modified.

The present disclosure describes an instrument, a method of using the instrument, and a manufacturing process for forming the instrument. In examples throughout the disclosure, the instrument is described as a medical instrument, and in particular a cutting device, for ease of explanation of the features of the contemplated embodiments. For example, the described medical instrument may be used to resect soft tissue or hard tissue, e.g., bone of a patient. By way of example, the described instrument may be coupled to or include a handle and/or a motorized device at a first end and may include a cutting tip or edge at the opposing end as described further below. The instrument may be wielded by a user at the first end to navigate the second end having the cutting edge to a desired target site, such as the surface of a bone, and to simultaneously control actuation of the second end.

While examples herein refer to the instrument as a medical instrument with a cutting edge, it should be understood and appreciated that the contemplated instrument is not limited to a cutting function and may be adapted to be an instrument for other applications, such as drill bits, drivers, shavers, burrs, chisels, or any other tool which is rotated about a central axis. Further, the instrument may be configured so that end pieces for different applications may be substituted for each other onto a shaft that receives such end pieces. Moreover, the instrument is not limited to medical applications, nor even to tools in general, as the structure of the contemplated instrument component connection can be applied to any two components which need to be detachably coupled to one another with axial and rotational fixation between components upon coupling.

In some arrangements, one or more objects are fabricated by additive manufacturing means, such as by an SLS, SLM, or EBM process. The materials used to form the one or more objects may be, but are not limited to being, metal powder. Such metal powder may be, in some arrangements, any one or any combination of titanium, titanium alloys such as but not limited to titanium, Ti-6Al-4V, stainless steel, cobalt chrome alloys, silver, tantalum and niobium.

In some embodiments, the one or more objects include a base formed in a plurality of layers. A first layer of the instrument is formed on a substrate by depositing and then selectively scanning with a high energy beam, e.g., a laser or electron beam, a first layer of powder to sinter or melt and thereby fuse selective portions of the first layer of powder together. Successive layers of the powder are then deposited and selectively scanned with the high energy beam, layer by layer, to sinter or melt and thereby fuse selective portions of each of the successive layers of powder together over the first layer to form the instrument. During this process, each layer of the instrument being formed is supported by one or both of the substrate and the previously scanned layers as the powder is heated to be fused together, and the formed portions of the base have continued support while cooling.

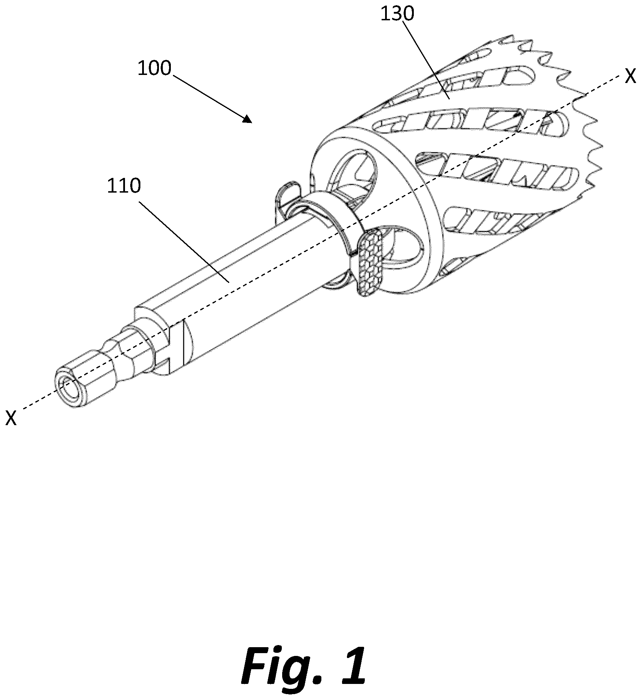

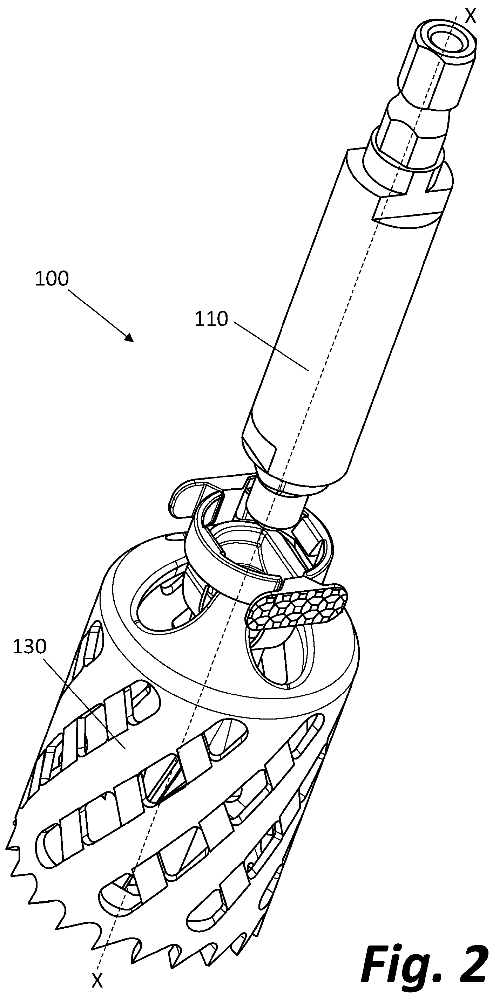

In a first aspect, the present disclosure relates to an instrument or parts of an instrument. Turning to the structure of the instrument, instrument 100 represents one embodiment and is shown in FIGS. 1 and 2. Instrument 100 includes a shaft 110 extending along a central longitudinal axis X and an extension piece 130 detachably coupleable to shaft 110. In general, the instrument of the depicted embodiment functions as a cutting end of a tool, where a proximal end of shaft opposite the extension piece may attach to additional components to define a complete instrument, such as an actuation device having a chuck for gripping an end of shaft 110 that is opposite the extension piece. FIG. 1 illustrates shaft 110 coupled to extension piece 130, whereas FIG. 2 illustrates shaft 110 decoupled from extension piece 130.

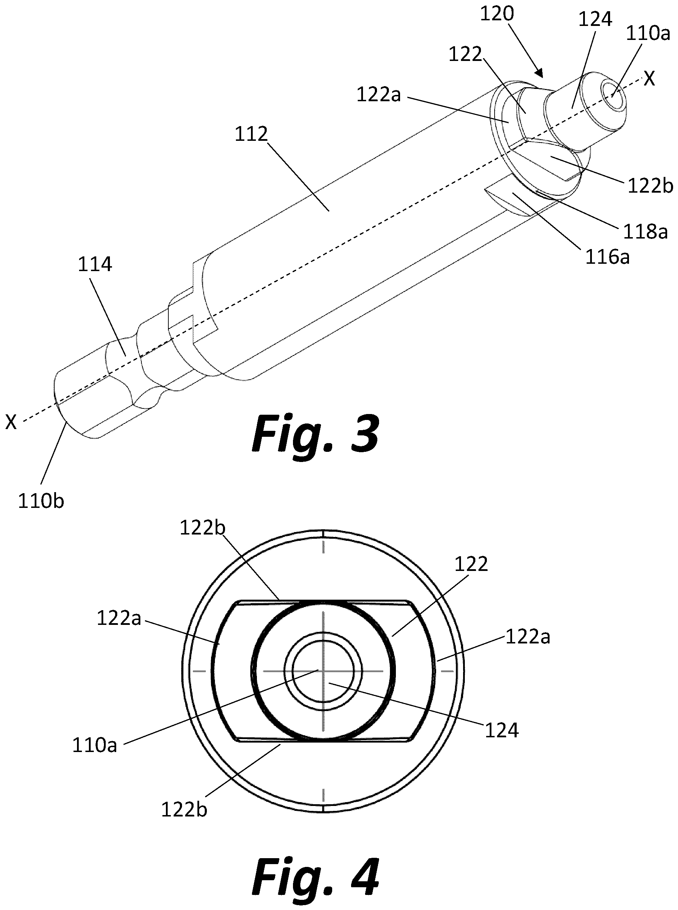

The structure of shaft 110 is shown in isolation in FIGS. 3 and 4. Shaft 110 extends between a first end 110a and a second end 110b opposite the first end along central longitudinal axis X. Shaft 110 includes interior segment 112, first end segment 120 extending from interior segment 112 toward first end 110a and second end segment 114 extending from interior segment 112 toward second end 110b. Second end segment 114 is sized and shaped to be coupled to a handle or motorized device to be wielded by a user and to apply torsion to shaft 110.

First end segment 120 of shaft 110 defines a cross-sectional dimension taken in any direction orthogonal to central longitudinal axis X. Similarly, interior segment 112 defines a cross-sectional dimension taken in any direction orthogonal to central longitudinal axis X. While the cross-sectional dimension varies along first end segment 120 as detailed below, the cross-sectional dimension of interior segment 112 adjacent to first end segment 120 is greater than or equal to all cross-sectional dimensions of end segment 120. First end segment 120 includes a neck portion 122 that extends from interior segment 112 to a head portion 124, and head portion 124 extends from neck portion 122 toward first end 110a. As shown in FIG. 4, neck portion 122 is shaped such that a cross-section of neck portion 122 taken along a plane orthogonal to central longitudinal axis X defines a generally elongate or oblong shape, *e.g.,* a stadium shape. That is, a first pair of opposing surfaces 122a on neck portion 122 are generally rounded and span a first distance along a side or surface of neck portion 122. A second pair of opposing surfaces 122b, which separate the first pair of opposing surfaces and are generally offset ninety degrees from the first pair of surfaces 122a, are generally straight or flat and span a second distance along a side or surface of neck portion 122 greater than the first distance. Head portion 124 extends to a free end of the shaft from the neck portion and is generally cylindrical in shape, having a diameter approximately equal to the distance between second pair of opposing surfaces 122b of neck portion 122.

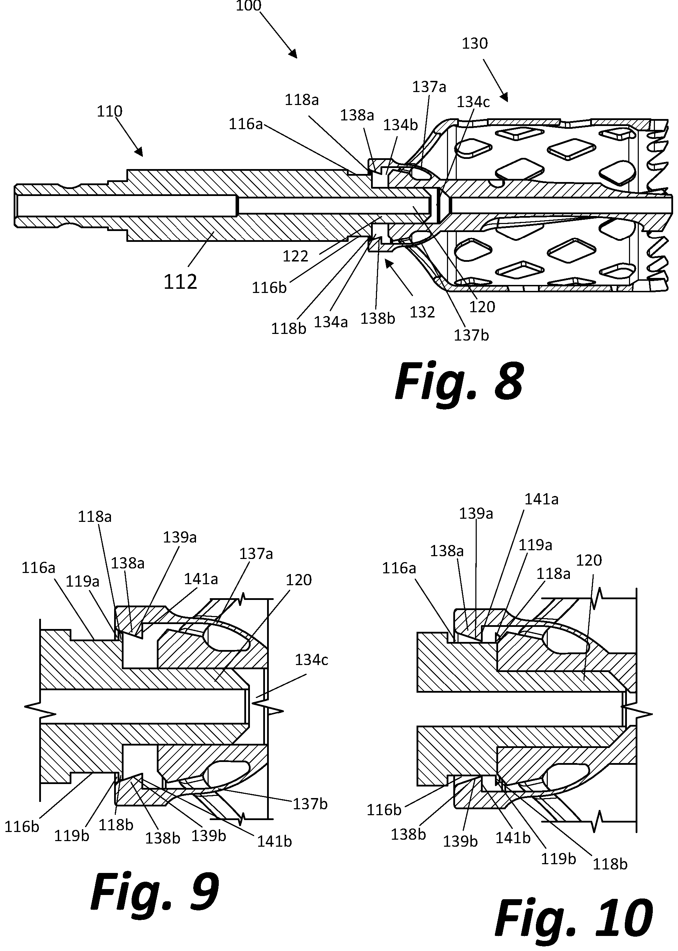

Interior segment 112 is generally cylindrical in shape and defines a pair of opposing recesses 116a, 116b positioned adj acent a first end of the interior segment 112 nearest first end 110a of instrument 100. Interior segment 112 further includes radial protrusions extending from respective surfaces of opposing recesses 116a, 116b at the first end of the interior segment 112 nearest first end 110a of shaft 110. These radial protrusions are in the form of lips 118a, 118b which extend radially beyond respective recessed portions 116a, 116b of interior segment 112 in the illustrated embodiment. While FIG. 3 only shows one lip 118a, it should be appreciated that a second lip 118b extends from the opposite recessed surface, as shown in FIGs. 8-10, for example.

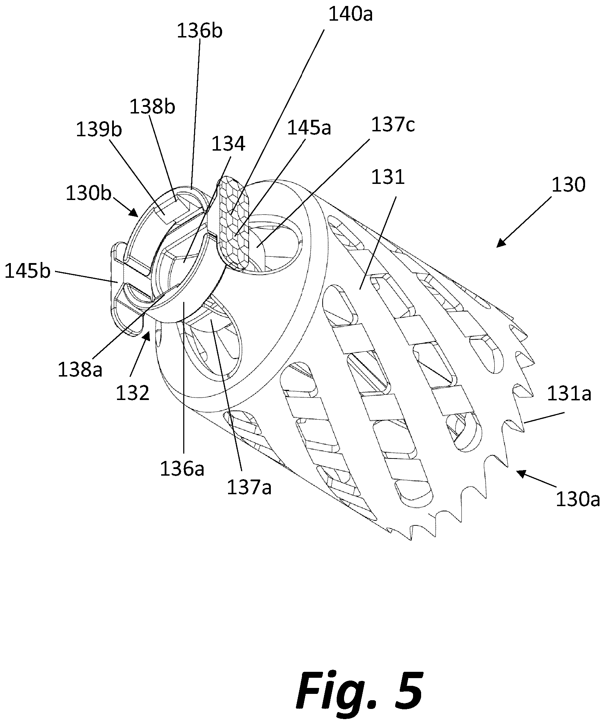

Extension piece 130 is shown in isolation in FIGS. 5-7. In the illustrated embodiment, extension piece 130 is a cutting instrument including a cutting element 131 having cutting edge 131a formed at a first end 130a of extension piece 130. Extension piece 130 further includes an attachment portion 132 extending from cutting element 131 to a second end 130b of extension piece 130 opposite first end 130a. Attachment portion 132 defines an opening 134 therein which is open to second end 130b.

Attachment portion 132 includes a pair of opposing arms 136a, 136b extending from cutting element 131 and surrounding opening 134. Each of the pair of arms 136a, 136b is rounded and substantially semi-circular about the central longitudinal axis X. Arms 136a, 136b have a length extending around a portion of a circumference of opening 134 and a depth in a direction parallel to central longitudinal axis X, wherein the length of each arm is greater than the depth. Further, each arm 136a, 136b has a thickness dimensioned such that the arms are rigid but still having a degree of flexibility and are thus capable of elastic deformation. Each arm 136a, 136b includes a connector piece 137a, 137b (as shown in FIGs. 5 and 8-9) extending from a center portion of the arm on a side of the arm facing first end 130a of extension piece 130 to an interior portion of attachment portion 132. The respective connector pieces securely couple the respective arms to the attachment portion. The connector pieces have a smaller thickness than the arms, which promotes greater flexibility in the connector pieces so that the connector pieces can be more easily deformed to move the arm when forces are applied to the attachment portion. On both sides of each connector piece 137a, 137b around the circumference of arms 136a, 136b, the arms are coupled directly to cutting element 131 for additional stability. Each of the pair of arms 136a, 136b includes a corresponding projection 138a, 138b extending radially inward from the arm toward opening 134. Each projection 138a, 138b extends from a corresponding arm 136a, 136b along a portion of the length of the arm and a substantial portion of the depth of the arm. As each projection 138a, 138b extends from a side of the arm adjacent second end 130b of extension piece toward the direction of first end 130a, the projection extends further toward the radial center of extension piece 130. In other words, a side of each projection 138a, 138b closer to first end 130a of extension piece 130 extends further radially inward than a side of the projection closer to second end 130b of extension piece, therefore defining ramped surfaces 139a, 139b on each corresponding projection.

Attachment portion 132 further includes a pair of opposing tabs 140a, 140b shown in FIGs. 5-7 that separate the respective arms of the pair of arms and are generally positioned ninety degrees offset from projections 138a, 138b about central axis X such that tabs 140a, 140b also surround opening 134 formed by attachment portion 132. As such, opening 134 is defined by the collective pair of opposing arms 136a, 136b and pair of opposing tabs 140a, 140b. Tabs 140a, 140b include generally elongate or stadium-shaped outer portions 145a, 145b that are oriented in a direction orthogonal to central longitudinal axis X and may have a knurled outer surface to promote ease of gripping by a user. An inner portion of each tab extends radially inward from the outer portion and is in operative contact with each arm 136a, 136b. The inner portion of tab 140a includes sloped surfaces 143a, 143b that extend radially inward from the outer portion of the tab such that the inner portion narrows in a radially inward direction. Similarly, tab 140b includes sloped surfaces 143c, 143d that extend radially inward from the outer portion of the tab such that the inner portion narrows in a radially inward direction. The inner portions of the respective tabs 140a, 140b are shown in FIGs. 5-7. Turning to the details of the operative contact between tabs 140a, 140b and arms 136a, 136b, in a neutral configuration, sloped surface 143a is proximate arm 136a, sloped surface 143b is proximate arm 136b, sloped surface 143c is proximate arm 140a and sloped surface 143d is proximate arm 136b. Similar to arms 136a, 136b, each of the pair of tabs 140a, 140b includes a connector piece 137c (the second connector piece for tab 140b is not shown) extending from a center portion of the tab on a side of the tab facing first end 130a of extension piece 130 to an interior portion of attachment portion 132, which securely couples the tabs to the attachment portion. Similar to connectors pieces 137a, 137b, the connector pieces coupled to each of the tabs have smaller thicknesses than the tabs which promote flexibility of the connector pieces and thereby promote movement of the tabs upon application of a force on the attachment portion.

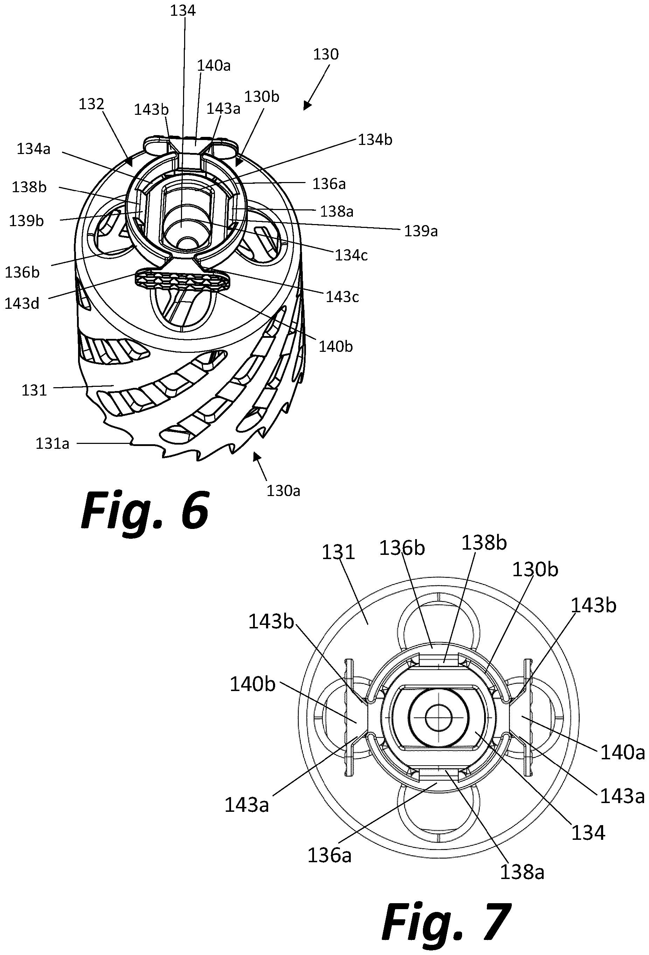

As shown in FIGs. 6 and 8, opening 134 is formed into three distinct portions: outer opening portion 134a, middle opening portion 134b, and inner opening portion 134c. Outer opening portion 134a is adjacent second end 130a of extension piece 130 (*e.g.,* adjacent the open end of opening 134) and defines the largest cross-sectional area of opening 134. Outer opening portion 134a is generally circular and defined by an inner surface of pair of arms 136a, 136b and pair of tabs 140a, 140b, with the exception that projections 138a, 138b extend inward into outer opening portion 134a. Adjacent to outer opening portion 134a is middle opening portion 134b which defines a smaller cross-sectional area than outer portion 134a. Middle portion 134b defines an elongate or oblong shape that complements neck portion 122 of end segment 120 as described above and further below. Inner opening portion 134c is adjacent to middle opening portion 134b and located on a side of middle portion 134b opposite outer portion 134a. Inner portion 134c defines a smaller cross-sectional area than outer portion 134a and middle portion 134a and is generally circular in shape, therefore defining a cylindrical opening portion. The complex structure defining opening 132 may result from fabricating the instrument monolithically using additive manufacturing techniques, as described further below.

In another aspect, the present disclosure relates to a method of assembling components of an instrument. In one embodiment, a method of assembly involves assembling shaft 110 and extension piece 130 of instrument 100. Shaft 110 and extension piece 130 are configured to detachably couple to one another, as illustrated in FIGS. 8-10 and described herein. That is, first end 110a of shaft 110 is coupled to second end 130b of extension piece 130a. End segment 120 of shaft 110 is sized and shaped to be inserted into opening 134 of attachment portion 132 to couple shaft 110 to extension piece 130. More specifically, head portion 124 of end segment 120 (or otherwise described as first end 110a of shaft 110) is the leading end of shaft 110 which corresponds to and mates with inner opening portion 134c defined by attachment portion 132. Further, neck portion 122 of end segment 120 follows head portion 124, corresponding to and mating with middle opening portion 134b (best shown in FIG. 6) defined by attachment portion 132. FIG. 10 illustrates when neck portion 122 is received at least in large part within middle opening portion 134b. It should be appreciated that head portion 124 is configured to mate with inner opening portion 134c regardless of the rotational orientation of shaft 110 and extension piece 130 due to corresponding cylindrical shapes. However, for the embodiment of instrument 100 specifically, due to the elongate shape of neck portion 122 and middle opening portion 134b, shaft 110 and extension piece 130 must be rotationally oriented so that the opposing surfaces 122b (shown in FIGS. 3 and 4) of neck portion 122 align with the flat sides bordering middle opening portion 134b to allow insertion therein.

As advancement continues, following end segment 120 of shaft 110 into opening 134 of extension piece 130 is interior segment 112 which is also inserted into opening 134 of attachment portion 132. The radial protrusions, or lips 118a, 118b, on respective recesses of the opposing pair of recesses 116a, 116b are positioned to correspond to outer opening portion 134a such that the lips and recesses are disposed within outer opening portion 134a when extension piece 130 is fully coupled to shaft 110. By orienting shaft 112 and extension piece 130 such that neck portion 122 is aligned with middle opening portion 134b, the components are positioned such that projections 138a, 138b of attachment portion 132 are aligned with recesses 116a, 116b of interior segment 112. As such, there are two orientations of the shaft with respect to the extension piece which would allow for insertion. Specifically, a second insertion orientation of the shaft is achieved when the shaft is rotated 180 degrees from ta first insertion orientation relative to the extension piece.

It should be appreciated that a diameter of interior segment (including lips 118a, 118b) is greater than a distance between a radially innermost edge of the first and second projections 138a, 138b when such projections are in a neutral state not subject to radially outward forces (*i.e.,* the distance between a radially innermost edge of one projection and the radially innermost edge of the other projection). Extension piece 130 is manufactured such that attachment portion 132 is elastically deformable, i.e., flexible. Thus, while attachment portion 132 is shown in a neutral, non-deformed state in FIGS. 5-7, attachment portion 132 may be slightly deformed during receipt of shaft 110 within opening 134 such that the generally circular opening 134 becomes more ovular. As such, during insertion of shaft 110 into extension piece 130, lips 118a, 118b are passed along a ramped surface of projection 138 (*e.g.,* ramps 139a, 139b shown in FIGS. 5-6 and 9-10) until lips 118a, 118b are passed internally beyond projections 138a, 138b. As lips 118a, 118b contact and slide along ramps 139a, 139b formed on the inner surfaces of projections 138a, 138b, arms 136a, 136b deform by spreading apart along an axis passing through projections 138a, 138b, the axis being orthogonal to central longitudinal axis X. With arms 136a, 136b spread apart, interior segment 112 is passable beyond projections 138a, 138b and into opening 134. Once lips 118a, 118b are passed completely beyond projections 138a, 138b and are seated between the projections and an interior end of opening 134, as shown in FIG. 10, attachment portion 132 is returned to its neutral, non-deformed state.

In the configuration shown in FIG. 10, extension piece 130 is coupled to shaft 110 and is in a fully inserted state within shaft 110. Further, lips 118a, 118b are nested between projections 138a, 138b and a surface separating openings 134a and 134b thereby preventing accidental detachment of shaft 110 from extension piece 130. Specifically, when in the fully inserted state (also referred to as the coupled or engaged configuration) as described above, an inner surface 141a, 141b of each projection 138a, 138b (*e.g*., surfaces facing away from the open end of opening 134) extend into corresponding recesses 116a, 116b and may contact a side surface 119a, 119b of each lip 118a, 118b (*e.g.,* a surface facing toward second end 110b of shaft 110) to prevent end segment 120 from backing out of opening 134, thereby axially retaining a portion of shaft 110 within extension piece 130. Although FIG. 10 shows a small distance between each lip 118a, 118b and the corresponding projection 138a, 138b, such a distance would not be present in a preferred embodiment. That is, each lip 118a, 118b would abut the corresponding projection 138a, 138b on one side of each lip while also abutting extension piece 130 on the opposing side of each lip to thereby prevent axial movement of shaft 110 relative to extension piece 130. Furthermore, the elongate shapes and mating of neck portion 122 with middle opening portion 134b prevents rotation of extension piece 130 relative to shaft 110. Therefore, when in the inserted state, extension piece 130 is axially retained to shaft 110 and rotationally fixed to shaft 110. As shown in FIG. 8, both shaft 110 and extension piece 130 are cannulated such that a cannulation extends through an entire length of instrument 100. In some exemplary surgical applications, such cannulation allows instrument 100 to be advanced over and along a guidewire when in use to improve the accuracy of a planned cut.

As noted above, extension piece 130 is detachably coupled to shaft 110 such that extension piece 130 may be decoupled or disengaged from shaft 110 after shaft reaches the fully inserted position. To expand opening 134 in order to remove shaft 110 from extension piece 130, opposing tabs 140a, 140b, shown in FIG. 6 for example, are pressed radially inward or toward each other, e.g., by a user of the instrument. By pressing the tabs in such manner, sloped surfaces 143a, 143b on the tabs press against ends of the respective arms of the pair of arms, causing the arms to move apart from each other, thereby increasing a size of outer opening 134a. As the arms move apart, projections 138a, 138b extending therefrom move apart in tandem such that the distance between the projections 138a, 138b increases. When the shaft 110 is disposed at this time, as it was in the example hereinbefore described, a space between projections 138a, 138b is sufficient so that lips 118a, 118b may be axially withdrawn between the projections to thereby withdraw shaft 110 entirely from extension piece 130.

Based on the detachable connection described above, it is contemplated that in other embodiments, various extension pieces having an attachment portion similar to attachment portion 132 may be used interchangeably with a single shaft 110 (and vice versa). It is also contemplated that various sizes of extension pieces or extension pieces with differing cutting features may be used interchangeably with a single shaft so long as the attachment portions of each of the differently sized extension pieces remain about the same size.

In another aspect, the present disclosure relates to a kit including various combinations of components that may be assembled into an instrument. In some embodiments, at least one shaft 110 and at least one extension piece 130, or various shafts and various extension pieces of similar or different sizes, may be provided together in a kit for selection by a user according to which size is appropriate for the scenario. The quick and convenient connection mechanism allows for an extension piece to be easily swapped out for another extension piece should there be an issue with the initial extension piece or desire for a clean piece. Furthermore, at least one shaft 110 and extension piece 130 may be provided in a kit with any combination of other tools and instruments with which instrument 100 may be used. For instance, a kit may include instrument 100 along with a handheld or motorized device to which instrument 100 may be coupled, or any combination of the above-noted components.

It is contemplated that variations of the illustrated embodiment are possible while still accomplishing the benefits of the modular instrument and the connection formed therein. For example, neck portion need not have the exact cross-sectional stadium-shape as illustrated, but may instead be any non-circular shape which will prevent rotation of extension piece 130 relative to shaft 110, such as a rectangle, triangle, *etc.,* so long as the shape of middle opening portion 134b defined by attachment portion 132 corresponds to and is able to mate with neck portion 122. For instance, neck portion may further be "X" shaped or "plus" shaped, having a cross-section formed by two parallelograms or rectangles intersecting at a common center portion of each. In such an arrangement, the extension piece would still be rotationally fixed to the shaft and would also allow for the shaft to be coupled to the extension piece in two orientations 180 degrees apart. Further in embodiments having an "X" or "plus" shaped neck portion, the interior segment of the shaft may define four recessed surfaces and lips equally spaced 90 degrees apart from one another around the circumference of the shaft, in which case there would be four orientations of the extension piece relative to the shaft in which the two parts can be coupled to one another, each orientation achieved by rotating the extension piece 90 degrees from the previous orientation relative to the shaft. In alternative embodiments, it may be desirable for certain applications to fix shaft 110 to extension piece 130 only axially and permit relative rotational movement, in which case neck portion 122 and middle opening portion 134b may both have circular cross-sections and the lips and recesses defined by the shaft may form a single lip/recess extending around the entire circumference of the shaft to allow for rotation of the extension piece with respect to the shaft. In other examples, a cross-sectional shape of the lip need not be rounded, but may take the form of any cross-sectional shape extending radially outward beyond the corresponding recess so long as it extends beyond the inner surface and the protrusion and fits within the outer opening of the extension piece, such as a triangular shape, a plurality of triangular shapes, a rectangular shape, etc. The same can be true of the shape of projections 138a, 138b - any shape may be used for the projections so long as the projections extend into the recesses defined by the shaft and prevent backout of the shaft when the shaft is coupled to the extension piece, such as a triangular shape, a plurality of triangular shapes, a round shape, etc. In some examples, the pair of arms may have a different curved shape than the illustrated semi-circular shape, such as an ovular shape, or alternatively a shape having a series of flat surfaces forming angles with respect to each other. In some examples, the extension may be symmetrical about certain planes. For example, in the illustrated example, the extension piece 130 may be symmetrical about a first plane extending through central longitudinal axis X and a center of the pair of tabs 140a, 140b. Extension piece 130 may also be symmetrical about a second plane orthogonal to the first plane noted above, wherein the second plane extends through central longitudinal axis X and a center of projections 138a, 138b.

As described above, extension piece 130 need not be a cutting tool having cutting element 131 exactly as illustrated herein. Instead, cutting element 131 may have any other shape, size, or configuration, so long as the element includes a similar attachment portion 132 as described herein for connection to shaft 110. Moreover, the extension piece may include attachment portion 132 extending from any other type of tool, device or component that is configured for rotation and to be coupled to a shaft, such as a drill, burr, sanding device, *etc.* In some embodiments, the shaft and extension piece may be solid all the way through and/or may not be cannulated as shown in FIG. 8.

Instrument 100 may be formed of titanium, steel such as 3164 steel/stainless steel, titanium or steel alloys, or other materials, which would allow for an appropriate degree of flexibility within certain parts such as the connector pieces of extension piece 130. In other embodiments, instrument 100 may be formed of plastic or other polymers, which may be advantageous for lower-torque applications, decreasing the weight or cost of the instrument and/or employing as single-use or disposable instruments. Shaft 110 may be manufactured using conventional manufacturing techniques such as laser-cutting or otherwise removing material from a workpiece to form a finished part. Extension piece 130 may be manufactured using additive manufacturing techniques. It is, however, contemplated that shaft 110 could be additively manufactured as well so that both shaft 110 and extension piece 130 are additively manufactured. Dimensions of the shaft and extension piece may be determined so that the shaft particularly corresponds with the extension piece to form a connection as described throughout the disclosure.

In another aspect, the present disclosure relates to a method of manufacturing a surgical instrument, such as instrument 100. In one embodiment, extension piece 130 is formed layer-by-layer using an additive layer manufacturing (ALM), *i.e.,* 3D printing process so that the extension piece may be formed monolithically without the need for welding together different structural features. Specifically, the extension piece that is formed may be monolithic in that cutting element 131 and attachment portion 132 are both part of an integrally formed structure. Thus, via ALM, extension piece 130 may be formed in a single stage process. Employment of an additive manufacturing process is particularly advantageous in reproducing the deformable features of the instrument component to a high degree of accuracy relative to a specified design. In some examples, the contemplated ALM processes may be powder-bed based and involve one or more of Selective Laser Sintering ("SLS"), Selective Laser Melting ("SLM"), Fused Deposition Modeling ("FDM"), Electron Beam Melting ("EBM"), Shape Deposition Manufacturing ("SDM"), Selective Laser Power Processing ("SLPP"), Direct Metal Laser Sintering ("DMLS"), Selecting Heating Sintering ("SHS"), material jetting, binder jetting, or another appropriate 3D printing technology as disclosed in U.S. Pat. Nos. 7,537,664; 8,590,157; 8,728,387; 9,180,010; 9,456,901, and U.S. Pat. Appl. Publ. No. 2021/0077268, the disclosures of which are hereby incorporated by reference herein in their entireties. When employing powder-bed based technologies, articles are produced in layer-wise fashion according to a predetermined digital model of such articles by heating, e.g., using a laser or an electron beam, multiple layers of powder, which may be a metallic powder, that are dispensed one layer at a time. The powder is sintered in the case of SLS technology and melted in the case of SLM technology, by the application of laser energy that is directed in raster-scan fashion to portions of the powder layer corresponding to a cross-section of the article. After the sintering or melting of the powder on one particular layer, an additional layer of powder is dispensed, and the process repeated, with sintering or melting taking place between the current layer and the previously laid layers until the article is complete. The powder layers similarly may be heated with EBM technology. Additive manufacturing techniques such as the ALM processes described above may be employed to form the instrument, including the extension piece and/or the shaft. It should also be appreciated that other devices, instruments, or components therefor that are contemplated by this disclosure may also be formed through an ALM process. In some instances, materials for one layer may be different than the materials for successive layers.

Formation of extension piece 130 using additive manufacturing allows for the construction of attachment portion 132 defining the complex opening 134 and all opening portions 134a, 134b, 134c as described above as a unitary piece, which would not otherwise be possible using other methods of manufacture. Instead of requiring additional machining or components after the initial manufacturing process as in conventional methods, the presently described instrument offers a single component design which can be more efficiently and effectively formed in one single process.

Extension piece 130 may be additively manufactured in any orientation. For example, the AM process may form the extension piece 130 so that the structure formed is oriented vertically relative to a table surface such that either first end 130a is face down and nearest a build plate used for the manufacture and cutting element 131 is formed prior to attachment portion 132, or second end 130b is face down and nearest the build plate so that attachment portion 132 is formed prior to cutting element 131. In other examples, extension piece 130 may be manufactured on its side in a horizontal orientation such that first end 130a and second end 130b are formed at least in part simultaneously. In one embodiment of forming extension piece 130, a first layer of metal powder may be deposited on a build plate of the manufacturing machine and that first layer of powder is selectively scanned by an electron beam to form a first portion nearest the build plate, which supports subsequent build layers as they are formed directly on top of the initial layer.

In some embodiments, the method of additively manufacturing a three-dimensional object may be performed in a cycle. A cycle may begin with depositing a first layer of powder onto a build plate. The first layer of powder may be selectively scanned with a high energy beam such as a laser or electron beam to sinter or melt the first layer of powder and to form initial portions of both the base of the object, e.g., attachment portion 132. After at least a first layer is scanned, successive layers of powder may be deposited and each such successive layer may be selectively scanned in a manner substantially similar to the first layer. The machine depositing the powder may be programmed to deposit the powder in locations corresponding to the shape of the three-dimensional object programmed into the machine (*e.g.,* extension piece 130). Additional layers may be deposited and scanned to form cutting element 131 on top of attachment portion 132 until extension piece 130 is fully formed, completing one full AM cycle.

In another aspect, the present disclosure relates to a method of using instrument 100 in a cutting process such as bone cutting in a surgical procedure. The contemplated instrument may be used in procedures including, for example, tissue repairs using formations of bone tunnels and implantation of suture anchors or shoulder arthroplasty surgery. In one specific example of shoulder surgery, the instrument may be used in a procedure to convert an anatomic shoulder to a reverse shoulder. In one embodiment, a method begins with selection of an extension piece for attachment to a shaft to assemble an instrument. In some examples, many different extension pieces may be available for attachment to the shaft. The chosen extension piece is then attached to the shaft as described elsewhere in the present disclosure. Surgical procedures are followed until a bone is prepared for cutting using the instrument. The instrument is then activated to drive an extension piece of the instrument along a planned cutting path to remove bone. When cutting is completed, the remaining steps of the surgery may proceed until completion. At any time after cutting, the extension piece may be removed from the shaft as described elsewhere in the present disclosure.

It is further contemplated that in some embodiments, instrument 100 as described herein may be used with or incorporated into a robotic system. For example, a robotic system may include one or more arms or shafts which may be operated by a robot that is either manually controlled by an operator or operated via the programming installed on the robot. To link the robot to the instrument, the instrument described in the present application may be coupled to an end effector of the robot. In some arrangements, the instrument may be manually installed onto the end effector of the robot either prior to or during a procedure. In other arrangements, the instrument may be handled and installed by the robot itself. Similarly, shaft 110 and extension piece 130 may be coupled decoupled in the manner described throughout this disclosure by the robot, and an alternative extension piece may be installed onto shaft 110 in the manner described during a procedure when desired, the maneuver being performed exclusively by the robot, whether it be programmed or controlled by an operator.

It is to be further understood that the present disclosure set forth herein contemplates embodiments that include any possible combination of the particular features set forth above, whether specifically disclosed herein or not. For example, where a particular feature is disclosed in the context of a particular aspect, arrangement, configuration, or embodiment, that feature can also be used, to the extent possible, in combination with and/or in the context of other particular aspects, arrangements, configurations, and embodiments of the present disclosure.

Although particular embodiments have been described herein, it is to be understood that these embodiments are merely illustrative of the principles and applications of the present disclosure. It is therefore to be understood that numerous modifications may be made to the illustrative embodiments and that other arrangements may be devised without departing from the spirit and scope of the present disclosure as defined by the appended claims.

## Claims

1. An instrument (100) comprising:
a shaft (110) including an interior segment (112) and an end segment (120) extending from the interior segment (112), the interior segment (112) having a first outer cross-sectional dimension and the end segment (120) having a second outer cross-sectional dimension less than the first outer cross-sectional dimension, the interior segment (112) including a radial protrusion (118a, 118b) adjacent to the end segment (120) such that the radial protrusion (118a, 118b) extends further from a central longitudinal axis (X) of the shaft (110) than an outer surface of the interior segment (112), wherein the first and second outer cross-sectional dimensions are measured orthogonally relative to the central longitudinal axis (X); and
an extension piece (130) including an attachment portion (132) removably engageable with the end segment (120) of the shaft (110), the attachment portion (132) including an opening (134) to receive the end segment (120), the opening (134) being defined in part by a pair of arms (136a, 136b), each arm of the pair of arms (136a, 136b) including a projection (138a, 138b) into the opening (134),
wherein the pair of arms (136a, 136b) are elastically deformable such that when the shaft (110) is inserted into the opening (134) of the extension piece (130) and the radial protrusion (118a, 118b) passes the projections (138a, 138b) of the pair of arms (136a, 136b), the pair of arms (136a, 136b) are pushed apart from a neutral position, and
wherein when the radial protrusion (118a, 118b) is in between the projections (138a, 138b) and an interior end of the opening (134), the projections (138a, 138b) are in the neutral position and prevent back out of the shaft (110) from the extension piece (130).

2. The instrument (100) of claim 1, wherein the extension piece (130) includes a cutting edge (131a) separate from the attachment portion (132).

3. The instrument (100) of one of claims 1 or 2, wherein the extension piece (130) is formed monolithically.

4. The instrument (100) of any one of the preceding claims, wherein the projection (138a) of a first arm (136a) of the pair of arms (136a, 136b) is disposed opposite the projection (138b) of a second arm (136b) of the pair of arms (136a, 136b) such that the projections (138a, 138b) of the first and second arms (136a, 136b) are separated by the opening (134) and face each other.

5. The instrument (100) of any one of the preceding claims, wherein the attachment portion (132) further comprises a pair of tabs (140a, 140b) that further define the opening (134), and wherein the pair of tabs (140a, 140b) are positioned opposite each other and are separated by the opening (134), the pair of tabs (140a, 140b) separating the pair of arms (136a, 136b).

6. The instrument (100) of claim 5, wherein the pair of tabs (140a, 140b) are offset from the pair of arms (136a, 136b) by ninety degrees around the opening (134) formed by the attachment portion (132).

7. The instrument (100) of one of claims 5 or 6, wherein the pair of tabs (140a, 140b) are positioned around the opening (134) such that when a radially inward pressure is applied to the pair of tabs (140a, 140b), the pair of tabs (140a, 140b) move inward and cause a radially outward deformation of the pair of arms (136a, 136b).

8. The instrument (100) of any one of the preceding claims, wherein when the radial protrusion (118a, 118b) is in between the projections (138a, 138b) and the interior end of the opening (132), the extension piece (130) is rotationally fixed to the shaft (110).

9. The instrument (100) of any one of the preceding claims, wherein the end segment (120) includes a neck (122) extending from the interior segment (112) having outer cross-sectional dimensions smaller than the first outer cross-sectional dimension of the interior segment (112).

10. The instrument (100) of claim 9, wherein the attachment portion (132) of the extension piece (130) defines a bore (134b) formed by surfaces that complement surfaces of the neck (122) of the end segment (120).

11. The instrument (100) of claim 10, wherein a cross-section of the neck (122) of the end segment (120) along a plane orthogonal to the central longitudinal axis (X) defines an oblong shape such that when the neck (122) is received within the bore (134b) of the extension piece (130), rotational movement of the extension piece (130) relative to the end segment (120) is prevented.

12. The instrument (100) of any one of claims 9-11, wherein the end segment (120) includes a head (124) extending from the neck (122) and the head (124) is cylindrical.

13. The instrument (100) of any one of the preceding claims, wherein the interior segment (112) is cylindrical and defines a pair of opposing recesses (116a, 116b) at an end of the interior segment (112) adjacent to the radial protrusion (118a, 118b).

14. The instrument (100) of any one of the preceding claims, wherein the radial protrusion (118a, 118b) includes an opposing pair of lips (118a, 118b) on the interior segment (112), the opposing pair of lips (118a, 118b) being adjacent to the end segment (120).

15. The instrument (100) of claim 14, wherein each of the opposing pair of lips (118a, 118b) is adjacent to one of a corresponding opposing pair of recesses (116a, 116b) defined on the interior segment (112) such that each one of the pair of lips (118a, 118b) extends radially outward beyond the corresponding recess (116a, 116b).
